# EUROPEAN PATENT SPECIFICATION

(11) **EP 3 285 758 B1**
(45) Date of publication and mention of the grant of the patent: **08.04.2020**
(21) Application number: 16783891.1
(22) Date of filing: 21.04.2016
(51) Int. Cl.: A61K 31/282, A61P 13/08, A61P 19/02, C07F 15/00

(54) **CO-CRYSTAL COMPOSITION AND ITS PHARMACEUTICAL USE**
CO-KRISTALL-ZUSAMMENSETZUNG UND DEREN PHARMAZEUTISCHE VERWENDUNG
COMPOSITION DE CO-CRISTAUX ET SON UTILISATION PHARMACEUTIQUE

(30) Priority: 22.04.2015 US 201562151113 P
(43) Date of publication of application: 28.02.2018
(73) Proprietor: Syn-Nat Products Enterprise LLC, Potomac, MD 20854 (US)
(72) Inventor: LIU, Xiaozhong, Potomac, MD 20854 (US)
(74) Representative: KATZAROV S.A.
(86) International application number: PCT/US2016/028720
(87) International publication number: WO 2016/172393

(56) References cited:
- US-A1- 2007 197 517
- LI, GQ ET AL.: 'Effect of Dicycloplatin, a Novel Platinum Chemotherapeutical Drug, on Inhibiting Cell Growth and Inducing Cell Apoptosis.' PLOS ONE. vol. 7, no. ISSUE, 2012, XP055325109
- OMAR, EK ET AL.: 'Does the Key to Treat Rheumatoid Nodules Lie with Oncology? - Is Cisplatin an Option?' BIUMED CENTRAL MUSCULOSKELETAL UISORDERS. vol. 14, no. SUPPL., 14 February 2013, page 1, XP021139262
- KREINER, B ET AL.: 'Neuroendocrine Carcinoma of the Seminal Vesicles Presenting with Lambert Eaton Syndrome: a Case Report.' JOURNAL OF MEDICAL CASE REPORTS. vol. 4, no. 1, 2010, pages 1 - 4, XP021082632
- LI, S ET AL.: 'Phase I Clinical Trial of the Novel Platin Complex Dicycloplatin: Clinical and Pharmacokinetic Results.' INTERNATIONAL JOURNAL OF CLINICAL PHARMACOLOGY AND THERAPEUTICS. vol. 51, no. 2, 2013, pages 96 - 105, XP055495942

## Description

### FIELD OF THE INVENTION

The present invention relates to dicycloplatin (DCP) for the prophylaxis or treatment of proliferative diseases, degenerative diseases, immunological diseases such as autoimmune diseases, and other diseases. Methods using the co-crystal, either alone or in combination with at least one additional therapeutic agent or adjuvant therapy agent, are disclosed.

### BACKGROUND OF THE INVENTION

Though platin has therapeutic effects in some cancers such as genitourinary cancer, nasopharyngeal cancer, cephalocircular cancer and lung cancer, this drug also shows high toxicity and severe side effects. Clinical studies revealed undesirable effects such as nephrotoxicity, neurotoxcity, ototoxicity, nausea, and vomiting, severely limiting the dosage and long term use of platin. Since B. Rosenber identified antitumor effect of *cis*-dichlorodiaminoplatin in 1969, cisplatin has been used widely in clinical medicine as an antitumor drug of platin analogues. Rosenberg et al. Nature, 1965, 205: 698; Nature, 1972, 222: 385. Later on, researchers discovered carboplatin, one of the second-generation antitumor drugs of platin analogues, which has an antitumor spectrum similar to cisplatin and a cross drug-resistance. Although the toxicity and side effects of carboplatin are significantly less than that of cisplatin, carboplatin also shows somewhat inferior therapeutic effects and there still exists side effects such as myelosuppression. In addition, carboplatin is not stable in aqueous solution. Therefore, it is desirable to identify antitumor pharmaceutics of platin analogues with superior effect, low toxicity and broad-spectrum.

Dicycloplatin (DCP) is a super molecule composed of carboplatin (CBP) and 1,1-cycyclobutane dicarboxylate (CBDCA) joined by strong hydrogen bonds. The solubility and stability of platinum complexes have a direct bearing on their activity, toxicity and pharmacokinetics. Preclinical studies have shown that DCP overcomes the problem of CBP instability in aqueous solution and maintains anticancer effects. Clinical evaluation in a Phase I dose-escalation study in patients with tumors showed that DCP was tolerated at doses ranging from 100 to 550 mg/m² and had potential efficacy in Chinese cancer patients. DCP showed favorable bioavailability and stability *in vivo*, and the recommended Phase II dosage for DCP-containing chemotherapy is 450 mg/m². DCP is currently being investigated as a monotherapy in several cancer types, such as prostatic carcinoma, and in combination with paclitaxel in a Phase II non-lung cancer study. Chemical structure of DCP is shown as formula I:

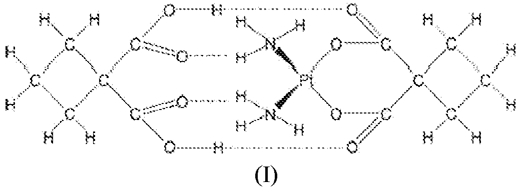

Although some studies demonstrated certain anti-tumor effects of DCP, the specific effects on particular cancers are not clearly illustrated. Moreover, no study showed the effects of DCP on other diseases. The current invention demonstrates DCP efficacy in the prophylaxis or treatment of several types of proliferative diseases, degenerative diseases, immunological diseases, and other diseases.

### SUMMARY OF THE INVENTION

The invention is as defined in the appended claims. The present invention relates to the pharmaceutical use of dicycloplatin (DCP) in the prophylaxis or treatment of proliferative diseases, degenerative diseases, immunological diseases such as but not limited to autoimmune diseases, and other diseases. In particular, the present invention related to a method of treating or preventing a disease in a subject, comprising administering a pharmaceutical composition comprising DCP to the subject, wherein the disease is a proliferative disease, a degenerative disease, or an immunological disease. In some embodiments, the pharmaceutical composition consists of DCP; in other embodiments, the pharmaceutical composition comprises DCP. In some embodiments, the pharmaceutical composition comprises an effective amount of DCP and at least one additional therapeutic agent or adjuvant therapy agent.

In one aspect, the present invention relates to the use of DCP in the prophylaxis or treatment of a proliferative disease, a disease caused by or related to excessive proliferation of cells and turnover of cellular matrix. In some embodiments, the proliferative disease may include but not be limited to: cancer, atherosclerosis, rheumatoid arthritis, psoriasis, idiopathic pulmonary fibrosis, scleroderma and cirrhosis of the liver. In some embodiments, the invention relates to a method of treating or preventing a proliferative disease in a subject, comprising administering a pharmaceutical composition comprising an effective amount of DCP to the subject.

In another aspect, the present invention relates to the use of DCP in the prophylaxis or treatment of a degenerative disease, a disease caused by or related to of a continuous process of cell degeneration. In some embodiments, the degenerative disease may include but not be limited to: Alzheimer's diseases, Amyotrophic Lateral Sclerosis (ALS), osteoarthritis, atherosclerosis, cancer, Charcot Marie Tooth disease (CMT), chronic obstructive pulmonary disease (COPD), chronic traumatic encephalopathy, diabetes, Ehlers-Danlos syndrome, essential tremor, Friedreich's ataxia, heart disease, Huntington's disease, inflammatory bowel disease (IBD), keratoconus, keratoglobus, macular degeneration, Marfan's syndrome, multiple sclerosis, multiple system atrophy, muscular dystrophy, Niemann Pick disease, osteoporosis, Parkinson's disease, progressive supranuclear palsy, prostatitis, petinitis, pigmentosa, rheumatoid arthritis and Tay-Sachs disease. In some embodiments, the invention relates to a method of treating or preventing a degenerative disease in a subject, comprising administering a pharmaceutical composition comprising an effective amount of DCP to the subject. In one embodiment, the degenerative disease is rheumatoid arthritis.

In one aspect, the present invention relates to the use of DCP in the prophylaxis or treatment of an immunological disease, a disease caused by or related to a dysfunction of the immune system. In some embodiments, the immunological disease may include but not be limited to: lupus, severe combined immunodeficiency (SCID), DiGeorge syndrome, hyperimmunoglobulin E syndrome, gout, common variable immunodeficiency (CVID), graft-versus-host disease (GVHD), chronic granulomatous disease (CGD), Wiskott-Aldrich syndrome (WAS), coeliac disease, Sjogren gren's syndrome, Hashimoto's thyroiditis, Graves' disease, autoimmune lymphoproliferative syndrome (ALPS), hyper IgM syndrome, leukocyte adhesion deficiency (LAD), NF-κB Essential Modifier (NEMO) Mutations, X-linked agammaglobulinemia (XLA), X-linked lymphoproliferative disease (XLP), Ataxia-telangiectasia, seasonal allergy, mastocytosis, perennial allergy, anaphylaxis, food allergy, allergic rhinitis, and atopic dermatitis. In some embodiments, the invention relates to a method of treating or preventing an immunological disease in a subject, comprising administering a pharmaceutical composition comprising an effective amount of DCP to the subject.

In one particular embodiment, the invention relates to a method of treating or preventing rheumatoid arthritis in a subject, comprising administering a pharmaceutical composition comprising an effective amount of dicycloplatin (DCP) to the subject.

In one aspect, the present invention relates to the use of DCP in the prophylaxis or treatment of a prostate disease, a disease related to the prostate gland. In some embodiments, the prostate disease may include but not be limited to: prostatitis, benign prostatic hyperplasia (BPH), enlarged prostate and prostate cancer. In some embodiments, the invention relates to a method of treating or preventing a prostate disease in a subject, comprising administering a pharmaceutical composition comprising an effective amount of DCP to the subject.

In one particular embodiment, the invention relates to a method of treating or preventing prostatitis in a subject, comprising administering a pharmaceutical composition comprising an effective amount of DCP to the subject.

In one particular embodiment, the invention relates to a method of treating or preventing BPH in a subject, comprising administering a pharmaceutical composition comprising an effective amount of DCP to the subject.

In another aspect, administration of the pharmaceutical composition according to the present invention can be via any common route as long as the target issue is available via the route. Suitable routes may include oral, buccal, by inhalation spray, sublingual, rectal, transdermal, vaginal, transmucosal, topical, nasal or intestinal administration; parenteral delivery, including intramuscular, subcutaneous, intramedullary injections, as well as intrathecal, direct intraventricular, orthotopic, intrademal,, intraperitoneal, intravenous, intra-articular, intra-sternal, intra-synovial, intra-hepatic, intralesional, intracranial, intraperitoneal, intranasal, or intraocular injections or other modes of delivery. The preferred delivery route depends on the particular disease to be treated and the subject's specific conditions.

In yet another aspect, the amount of DCP in the pharmaceutical composition administered to a subject may be about 0.005 to 20 mg/kg body weight, about 0.005 to 10 mg/kg body weight, about 0.005 to 5 mg/kg body weight, about 0.005 to 2.5 mg/kg body weight, 0.01 to 20 mg/kg body weight, about 0.01 to 10 mg/kg body weight, about 0.01 to 5 mg/kg body weight, about 0.01 to 2.5 mg/kg body weight, 0.1 to 20 mg/kg body weight, about 0.1 to 10 mg/kg body weight, about 0.1 to 5 mg/kg body weight, or about 0.1 to 2.5 mg/kg body weight. The preferred amount of DCP depends on the particular disease to be treated and the subject's specific conditions.

In yet another aspect, the administration of the pharmaceutical composition comprising DCP may last at least 1, 2, 3, 4, 5, 6, 7, 8, 9, 10, 11, 12, 13, 14, 21, 28, 35, 42, 49, 56, 63, 70, 77, 84, 91 or 98 days. In one embodiment, the administering of the pharmaceutical composition comprising DCP may last at least one week. In one embodiment, the administering of the pharmaceutical composition comprising DCP may last at least two weeks. The preferred period of administration depends on the particular disease to be treated and the subject's specific conditions.

### BRIEF DESCRIPTION OF THE DRAWINGS

**Fig. 1** shows the inhibition of human fibroblast like synoviocytes rheumatoid arthritis (HFLS-RA) cell proliferation after treatment with DCP at different concentrations; DCP concentration in HFLS-RA: 1.563µg/ml; 3.125µg/ml; 6.25µg/ml; 12.5µg/ml; 25µg/ml; 50µg/ml; 100µg/ml; IC₅₀: 3.914µg/ml.
**Fig. 2** shows the inhibition of MH7A cell proliferation after treatment with DCP at different concentrations; DCP concentration in MH7A: 1.563µg/ml; 3.125µg/ml; 6.25µg/ml; 12.5µg/ml; 25µg/ml; 50µg/ml; 100µg/ml; IC₅₀: 8.926µg/ml.
**Fig. 3** shows the prostate tissues of a control mouse.
**Fig. 4** shows the prostate tissues of a mouse with prostatitis.
**Fig. 5** shows the prostate tissues of a prostatitis mouse after treatment with DCP for one week.
**Fig. 6** shows the prostate tissues of a control mouse, without benign prostatic hyperplasia (BPH).
**Fig. 7** shows the prostate tissue of a BPH mouse.
**Fig. 8** shows the prostate tissues of a BPH mouse treated by Finasteride (PROSCAR).
**Fig. 9** shows the prostate tissues of a BPH mouse treated by DCP injection for one week.
**Fig. 10** shows the prostate tissues of a BPH mouse treated by DCP administered orally.

### DETAILED DESCRIPTION OF THE INVENTION

Unless defined otherwise, all technical and scientific terms used herein have the same meaning as is commonly understood by one of skill in the art to which this invention belongs. All patents and publications referred to herein are incorporated by reference in their entireties.

The term "effective amount" or "therapeutically effective amount" refers to that amount of a compound or combination of compounds as described herein that is sufficient to effect the intended application including, but not limited to, prophylaxis or treatment of diseases. A therapeutically effective amount may vary depending upon the intended application (*in vitro* or *in vivo*), or the subject and disease condition being treated (e.g., the weight, age and gender of the subject), the severity of the disease condition, the manner of administration, etc. which can readily be determined by one of ordinary skill in the art. The term also applies to a dose that will induce a particular response in target cells and/or tissues (e.g., the reduction of cell proliferation and/or morphological alteration of the tissue). The specific dose will vary depending on the particular compounds chosen, the dosing regimen to be followed, whether the compound is administered in combination with other compounds, timing of administration, the tissue to which it is administered, and the physical delivery system in which the compound is carried.

A therapeutic "effect" as that term is used herein, encompasses a therapeutic benefit and/or a prophylactic benefit. A prophylactic effect (e.g. terms such as "prophylaxis," "prevent" and "reducing the likelihood for developing") includes delaying or eliminating the appearance of a disease or condition, delaying or eliminating the onset of symptoms of a disease or condition, slowing, halting, or reversing the progression of a disease or condition, or any combination thereof by administering a drug before the onset of the disease or condition. A treatment effect (e.g. with terms such as "treatment" and "treat") includes reducing or eliminating the appearance of a disease or condition, reducing or eliminating the symptoms of a disease or condition, slowing, halting, or reversing the progression of a disease or condition, or any combination thereof by administering a drug after the onset of the disease or condition.

A "subject" as the term is used herein, refers to a human or non-human animal. In some embodiments, the subject is a mammal. In some embodiments, the subject is human.

When ranges are used herein to describe, for example, physical or chemical properties such as molecular weight or chemical formulae, all combinations and subcombinations of ranges and specific embodiments therein are intended to be included. Use of the term "about" when referring to a number or a numerical range means that the number or numerical range referred to is an approximation within experimental variability (or within statistical experimental error), and thus the number or numerical range may vary. The variation is typically from 0% to 15%, preferably from 0% to 10%, more preferably from 0% to 5% of the stated number or numerical range. The term "comprising" (and related terms such as "comprise" or "comprises" or "having" or "including") includes those embodiments such as, for example, an embodiment of any composition of matter, method or process that "consist of' or "consist essentially of' the described features.

Compounds used in the present invention also include crystalline and amorphous forms of those compounds, including, for example, polymorphs, pseudopolymorphs, solvates, hydrates, unsolvated polymorphs (including anhydrates), conformational polymorphs, and amorphous forms of the compounds, as well as mixtures thereof. "Crystalline form" and "polymorph" are intended to include all crystalline and amorphous forms of the compound, including, for example, polymorphs, pseudopolymorphs, solvates, hydrates, unsolvated polymorphs (including anhydrates), conformational polymorphs, and amorphous forms, as well as mixtures thereof, unless a particular crystalline or amorphous form is referred to.

The present invention in various aspects and embodiments involves uses of DCP for the prophylaxis or treatment of various diseases and methods of treating or preventing the diseases by administering a pharmaceutical composition comprising DCP.

The diseases to be treated or prevented include but are not limited to proliferative diseases, degenerative diseases, immunological diseases, and other diseases. In some embodiments, the disease is rheumatoid arthritis, gout, lupus, osteoporosis, psoriasis, and other autoimmune diseases, chronic inflammatory proliferative diseases, benign prostatic hyperplasia (BPH), multiple sclerosis, vascular proliferative diseases or thyroid proliferative diseases.

In some embodiments, the administration of DCP treats or prevents the diseases by modulating the immunological reaction of the subject. Particularly in some embodiments, DCP enhances the immunological reaction and in other embodiments, DCP reduces the immunological reaction. For example, in some embodiments DCP enhances or reduces the number and/or effectiveness of T cells; in some embodiments DCP enhances or reduces the number and/or effectiveness of B cells. The capability to modulate the immunological reaction may affect the efficacy of DCP in treating and/or preventing the proliferative diseases, immunological diseases, degenerative diseases, and other diseases.

In some embodiments, the pharmaceutical composition may consist of DCP. In some embodiments, the pharmaceutical composition may comprise DCP and at least one additional therapeutic agent or adjuvant therapy agent. The additional therapeutic agent or adjuvant therapy agent may be selected from but is not limited to: folic acid, coenzyme Q10, curcumin, glutathione (GSH), aloe vera, oryzanol, 5-fluorouracil, bortezomib, or a combination thereof. Depending on the particular disease to be treated, the additional therapeutic agent or adjuvant therapy agent may include drugs already known. In some embodiments, the additional therapeutic agent or adjuvant therapy agent may include drugs that have already been clinically accepted to treat or prevent the disease.

In some embodiments, the pharmaceutical composition may comprise DCP and a pharmaceutically acceptable carrier or excipient. "Pharmaceutically acceptable carrier" or "pharmaceutically acceptable excipient" is intended to include any and all solvents, dispersion media, coatings, antibacterial and antifungal agents, isotonic and absorption delaying agents, and inert ingredients. The use of such pharmaceutically acceptable carriers or pharmaceutically acceptable excipients for active pharmaceutical ingredients is well known in the art. Except insofar as any conventional pharmaceutically acceptable carrier or pharmaceutically acceptable excipient is incompatible with the active pharmaceutical ingredient, its use in the therapeutic compositions of the invention is contemplated. Additional active pharmaceutical ingredients, such as other drugs, can also be incorporated into the described compositions and methods.

In some embodiments, the present invention provides a method of treating, preventing, reducing or alleviating the symptoms of, and/or slowing or halting the progress of rheumatoid arthritis in a subject in need thereof, the method comprising administrating to the subject an effective amount of a pharmaceutical composition comprising DCP. In one embodiment, the pharmaceutical composition consists of DCP. In some embodiments, the pharmaceutical composition further comprises at least one additional therapeutic agent or adjuvant therapy agent. In a specific embodiment, the additional therapeutic agent or adjuvant therapy agent may be selected from: folic acid, coenzyme Q10, curcumin, glutathione (GSH), aloe vera, oryzanol, 5-fluorouracil, and bortezomib. In one embodiment, the pharmaceutical composition comprises DCP and a pharmaceutically acceptable carrier or excipient.

In some embodiments, for prophylaxis or treatment of rheumatoid arthritis, the amount of DCP in the pharmaceutical composition administered to a subject may be about 0.005 to 20 mg/kg body weight, about 0.005 to 10 mg/kg body weight, about 0.005 to 5 mg/kg body weight, about 0.005 to 2.5 mg/kg body weight, 0.01 to 20 mg/kg body weight, about 0.01 to 10 mg/kg body weight, about 0.01 to 5 mg/kg body weight, about 0.01 to 2.5 mg/kg body weight, 0.1 to 20 mg/kg body weight, about 0.1 to 10 mg/kg body weight, about 0.1 to 5 mg/kg body weight, about 0.1 to 2.5 mg/kg body weight, 1 to 20 mg/kg body weight, about 1 to 10 mg/kg body weight, about 1 to 5 mg/kg body weight, or about 1 to 2.5 mg/kg body weight. In one embodiment, the amount of DCP is about 0.01 to 5 mg/kg body weight. In another embodiment, the amount of DCP is about 1 to 10 mg/kg body weight.

In one embodiment, for prophylaxis or treatment of rheumatoid arthritis, the pharmaceutical composition comprising the DCP is administered with injections or via the oral route. In one embodiment, for prophylaxis or treatment of rheumatoid arthritis, the pharmaceutical composition comprising the DCP is administered for at least one, two or three weeks.

In some embodiments, the present invention provides a method of treating, preventing, reducing or alleviating the symptoms of, and/or slowing or halting the progress of prostatitis in a subject in need thereof, the method comprising administrating to the subject an effective amount of a pharmaceutical composition comprising DCP. In one embodiment, the pharmaceutical composition consists of DCP. In some embodiments, the pharmaceutical composition further comprises at least one additional therapeutic agent or adjuvant therapy agent. In a specific embodiment, the additional therapeutic agent or adjuvant therapy agent may be selected from: folic acid, coenzyme Q10, curcumin, glutathione (GSH), aloe vera, oryzanol, 5-fluorouracil, and bortezomib. In one embodiment, the pharmaceutical composition comprises DCP and a pharmaceutically acceptable carrier or excipient.

In some embodiments, for prophylaxis or treatment of prostatitis, the amount of DCP in the pharmaceutical composition administered to a subject may be about 0.005 to 20 mg/kg body weight, about 0.005 to 10 mg/kg body weight, about 0.005 to 5 mg/kg body weight, about 0.005 to 2.5 mg/kg body weight, 0.01 to 20 mg/kg body weight, about 0.01 to 10 mg/kg body weight, about 0.01 to 5 mg/kg body weight, about 0.01 to 2.5 mg/kg body weight, 0.1 to 20 mg/kg body weight, about 0.1 to 10 mg/kg body weight, about 0.1 to 5 mg/kg body weight, or about 0.1 to 2.5 mg/kg body weight. In one embodiment, the amount of DCP is about 0.01 to 5 mg/kg body weight.

In one embodiment, for prophylaxis or treatment of prostatitis, the pharmaceutical composition comprising the DCP is administered with injections or via the oral route. In one embodiment, for prophylaxis or treatment of prostatitis, the pharmaceutical composition comprising the DCP is administered for at least one, two or three weeks.

In some embodiments, the present invention provides a method of treating, preventing, reducing or alleviating the symptoms of, and/or slowing or halting the progress of benign prostatic hyperplasia (BPH) in a subject in need thereof, the method comprising administrating to the subject an effective amount of a pharmaceutical composition comprising DCP. In one embodiment, the pharmaceutical composition consists of DCP. In some embodiments, the pharmaceutical composition further comprises at least one additional therapeutic agent or adjuvant therapy agent. In a specific embodiment, the additional therapeutic agent or adjuvant therapy agent may be selected from: folic acid, coenzyme Q10, curcumin, glutathione (GSH), aloe vera, oryzanol, 5-fluorouracil, and bortezomib. In one embodiment, the pharmaceutical composition comprises DCP and a pharmaceutically acceptable carrier or excipient.

In some embodiments, for prophylaxis or treatment of BPH, the amount of DCP in the pharmaceutical composition administered to a subject may be about 0.005 to 20 mg/kg body weight, about 0.005 to 10 mg/kg body weight, about 0.005 to 5 mg/kg body weight, about 0.005 to 2.5 mg/kg body weight, 0.01 to 20 mg/kg body weight, about 0.01 to 10 mg/kg body weight, about 0.01 to 5 mg/kg body weight, about 0.01 to 2.5 mg/kg body weight, 0.1 to 20 mg/kg body weight, about 0.1 to 10 mg/kg body weight, about 0.1 to 5 mg/kg body weight, or about 0.1 to 2.5 mg/kg body weight. In one embodiment, the amount of DCP is about 0.01 to 5 mg/kg body weight.

In one embodiment, for prophylaxis or treatment of BPH, the pharmaceutical composition comprising the DCP is administered with injections or via the oral route. In one embodiment, for prophylaxis or treatment of BPH, the pharmaceutical composition comprising the DCP is administered for at least one, two or three weeks.

In some embodiments, the present invention provides a method of treating, preventing, reducing or alleviating the symptoms of, and/or slowing or halting the progress of osteoarthritis and related collagen and/or auto-immune rheumatoid diseases in a subject in need thereof, the method comprising administrating to the subject an effective amount of a pharmaceutical composition comprising DCP. In one embodiment, the pharmaceutical composition consists of DCP. In some embodiments, the pharmaceutical composition further comprises at least one additional therapeutic agent or adjuvant therapy agent. In some embodiments, the pharmaceutical composition further comprises a pharmaceutically acceptable carrier or excipient. In one embodiment, the pharmaceutical composition comprising the DCP is administered with injections or via the oral route. In one embodiment, the pharmaceutical composition comprising the DCP is administered for at least one, two or three weeks.

In some embodiments, the present invention provides a method of treating, preventing, reducing or alleviating the symptoms of, and/or slowing or halting the progress of gout in a subject in need thereof, the method comprising administrating to the subject an effective amount of a pharmaceutical composition comprising DCP. In one embodiment, the pharmaceutical composition consists of DCP. In some embodiments, the pharmaceutical composition further comprises at least one additional therapeutic agent or adjuvant therapy agent. In some embodiments, the pharmaceutical composition further comprises a pharmaceutically acceptable carrier or excipient. In one embodiment, the pharmaceutical composition comprising the DCP is administered with injections or via the oral route. In one embodiment, the pharmaceutical composition comprising the DCP is administered for at least one, two or three weeks.

In some embodiments, the present invention provides a method of treating, preventing, reducing or alleviating the symptoms of, and/or slowing or halting the progress of psoriasis and other related diseases in a subject in need thereof, the method comprising administrating to the subject an effective amount of a pharmaceutical composition comprising DCP. In one embodiment, the pharmaceutical composition consists of DCP. In some embodiments, the pharmaceutical composition further comprises at least one additional therapeutic agent or adjuvant therapy agent. In some embodiments, the pharmaceutical composition may further comprise one or more anti-malarial drugs such as chloroquine phosphate, primaquine phosphate, such as hydroxychloroquine, pyrimethamine, quinine, artemisinin, artemether (Arteether), artesunate, and thalidomide. In some embodiments, the pharmaceutical composition further comprises a pharmaceutically acceptable carrier or excipient. In one embodiment, the pharmaceutical composition comprising the DCP is administered with injections or via the oral route. In one embodiment, the pharmaceutical composition comprising the DCP is administered for at least one, two or three weeks. In one embodiment, aerosol spray may also be used to treat the skin problems caused by psoriasis.

In some embodiments, the present invention provides a method of treating, preventing, reducing or alleviating the symptoms of, and/or slowing or halting the progress of Lupus Erythematosus (LE) and other related diseases in a subject in need thereof, the method comprising administrating to the subject an effective amount of a pharmaceutical composition comprising DCP. In one embodiment, the pharmaceutical composition consists of DCP. In some embodiments, the pharmaceutical composition further comprises at least one additional therapeutic agent or adjuvant therapy agent. In some embodiments, the pharmaceutical composition may further comprise one or more clinically approved immunosuppressive drugs (e.g., hydroxychloroquine and corticosteroids). In some embodiments, the pharmaceutical composition further comprises a pharmaceutically acceptable carrier or excipient. In one embodiment, the pharmaceutical composition comprising the DCP is administered with injections or via the oral route. In one embodiment, the pharmaceutical composition comprising the DCP is administered for at least one, two or three weeks. In one embodiment, aerosol spray may also be used to treat the skin problems caused by LE.

In some embodiments, the present invention provides a method of treating, preventing, reducing or alleviating the symptoms of, and/or slowing or halting the progress of multiple scerosis (MS) in a subject in need thereof, the method comprising administrating to the subject an effective amount of a pharmaceutical composition comprising DCP. In one embodiment, the pharmaceutical composition consists of DCP. In some embodiments, the pharmaceutical composition further comprises at least one additional therapeutic agent or adjuvant therapy agent. In some embodiments, the pharmaceutical composition further comprises a pharmaceutically acceptable carrier or excipient. In one embodiment, the pharmaceutical composition comprising the DCP is administered with injections or via the oral route.

In some embodiments, the present invention provides a method of treating, preventing, reducing or alleviating the symptoms of, and/or slowing or halting the progress of coeliac disease, Sjogren gren's syndrome, Hashimoto's thyroiditis, Graves' disease and other auto-immune diseases in a subject in need thereof, the method comprising administrating to the subject an effective amount of a pharmaceutical composition comprising DCP. In one embodiment, the pharmaceutical composition consists of DCP. In some embodiments, the pharmaceutical composition further comprises at least one additional therapeutic agent or adjuvant therapy agent. In some embodiments, the pharmaceutical composition may further comprise one or more anti-malarial drugs such as chloroquine phosphate, primaquine phosphate, such as hydroxychloroquine, pyrimethamine, quinine, artemisinin, artemether (Arteether), artesunate, and thalidomide. In some embodiments, the pharmaceutical composition further comprises a pharmaceutically acceptable carrier or excipient. In one embodiment, the pharmaceutical composition comprising the DCP is administered with injections or via the oral route. In one embodiment, aerosol spray may also be used to treat the skin problems caused by any of these diseases.

In some embodiments, the present invention provides a method of treating, preventing, reducing or alleviating the symptoms of, and/or slowing or halting the progress of graft-versus-host disease (GVHD) in a subject in need thereof, the method comprising administrating to the subject an effective amount of a pharmaceutical composition comprising DCP. GVHD is a complication following an allogeneic tissue transplant, commonly associated with stem cell or bone marrow transplant but also applies to other forms of tissue graft. GVHD can also occur after a blood transfusion if the blood products used have not been irradiated or treated with an approved pathogen reduction system. DCP suppresses the T-cell-mediated immune onslaught on the host tissues. It is desirable to taper off the post-transplant high-level steroid doses to lower levels, at which point the appearance of mild GVHD may be welcome, especially in HLA mis-matched patients, as it is typically associated with a graft-versus-tumor effect.

In some embodiments, DCP may be used for prophylaxis or treatment of GVHD. In one embodiment, the pharmaceutical composition consists of DCP. In some embodiments, the pharmaceutical composition further comprises at least one additional therapeutic agent or adjuvant therapy agent. In some embodiments, the pharmaceutical composition further comprises a pharmaceutically acceptable carrier or excipient. In one embodiment, the pharmaceutical composition comprising the DCP is administered with injections or via the oral route.

The following examples are provided to describe and illustrate the present invention. As such, they should not be construed to limit the scope of the invention. Those in the art will well appreciate that many other embodiments also fall within the scope of the invention, as it is described hereinabove and in the claims.

### Examples

The effects of DCP on various diseases can be demonstrated by results obtained from *in vivo* and *in vitro* studies and clinical trials.

### Clinical Trial Studies

DCP alleviated symptoms for clinical trial patients suffering from multiple diseases. In China, DCP (1 to about 10 mg/kg body weight) in combination with cisplatin and Paclitaxel were used in Phase II and Phase III clinical trials for treatment of patients having small cell lung cancer (SCLC). The cancer patients were simultaneously suffering from a number of proliferative diseases, degenerative diseases, immunological diseases, and other diseases as indicated above. For example, some of the patients were suffering from rheumatoid arthritis, osteoarthritis, arthrolithiasis (gout) and/or other diseases. The researchers found that the symptoms of all the diseases were reduced by the combination of DCP, ciplatin and Placlitaxel. However, patient being treated with the combination were also affected by strong side effects such as nephrotoxicity, neurotoxcity, ototoxicity, nausea, and vomiting. Treatment with DCP alone (1 to about 10 mg/kg body weight) for the same types of patient revealed that DCP significantly alleviated the symptoms of proliferative diseases, degenerative diseases, immunological diseases, and other diseases, even more effectively than the combination. Such diseases include at least rheumatoid arthritis, osteoarthritis, and arthrolithiasis (gout). In addition, less patients receiving DCP alone were suffering from side effects and the side effects were of less severity compared to the combination of DCP, cisplatin and Paclitaxel. When DCP was administered with an additional therapeutic agent or adjuvant therapy agent such as folic acid, coenzyme Q10, curcumin, glutathione (GSH), aloe vera, oryzanol, 5-fluorouracil, bortezomib, the effects to reduce disease symptoms were further enhanced.

### Studies on Rheumatoid Arthritis

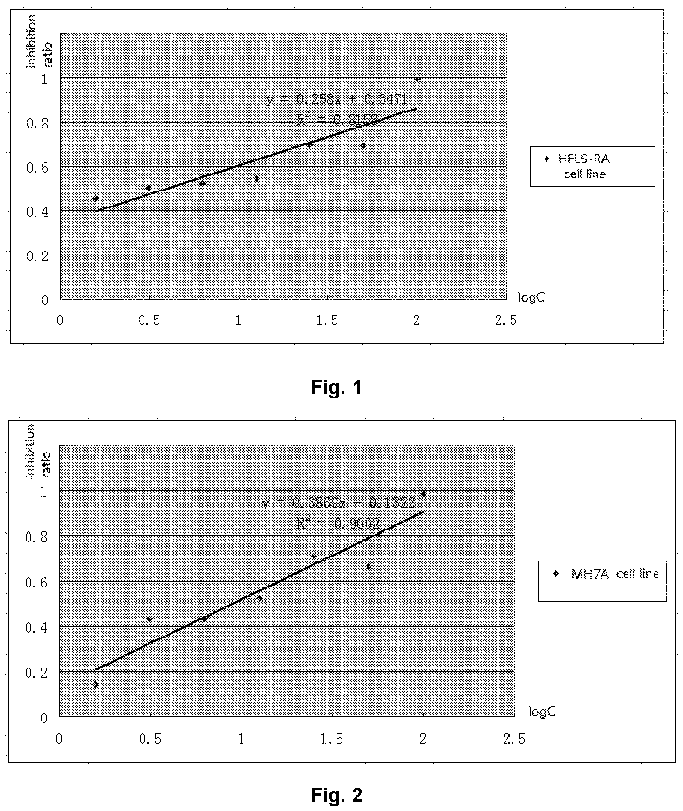

Human fibroblast-like synoviocytes rheumatoid arthritis (HFLS-RA) cells were cultured *in vitro* and proliferation of the cells was measured by quantifying cells numbers 48 hours after treatment. The cells were treated with DCP at concentrations of 1.563µg/ml; 3.125µg/ml; 6.25µg/ml; 12.5µg/ml; 25µg/ml; 50µg/ml; or 100µg/ml. DCP significantly reduced HFLS-RA proliferation with an IC₅₀ of 3.914µg/ml. The results are shown in Fig. 1.

Human MH7A synovial cells were cultured *in vitro* and proliferation of the cells was measured by quantifying cells numbers 48 hours after treatment. The cells were treated with DCP at concentrations of 1.563µg/ml; 3.125µg/ml; 6.25µg/ml; 12.5µg/ml; 25µg/ml; 50µg/ml; or 100µg/ml. DCP significantly reduced MH7A proliferation with an IC₅₀ of 8.926µg/ml. The results are shown in Fig. 2.

As shown in Fig. 1, 99% of HFLS-RA cell proliferation was inhibited by DCP (IC₅₀: 3.125µg/ml); as shown in Fig. 2, 99% of MH7A cell proliferation was inhibited by DCP ((IC₅₀: 8.92µg/ml)). The results confirmed the anti-proliferation efficacy of DCP in rheumatoid arthritis.

### Studies on Prostatitis

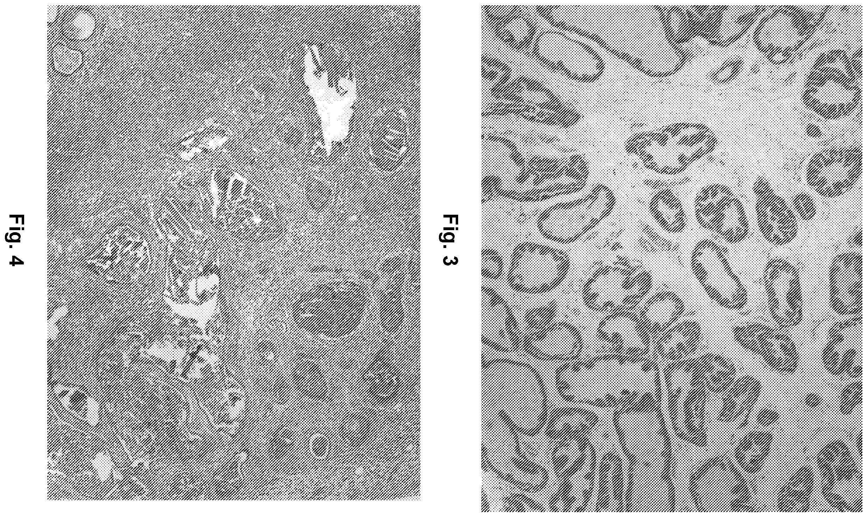

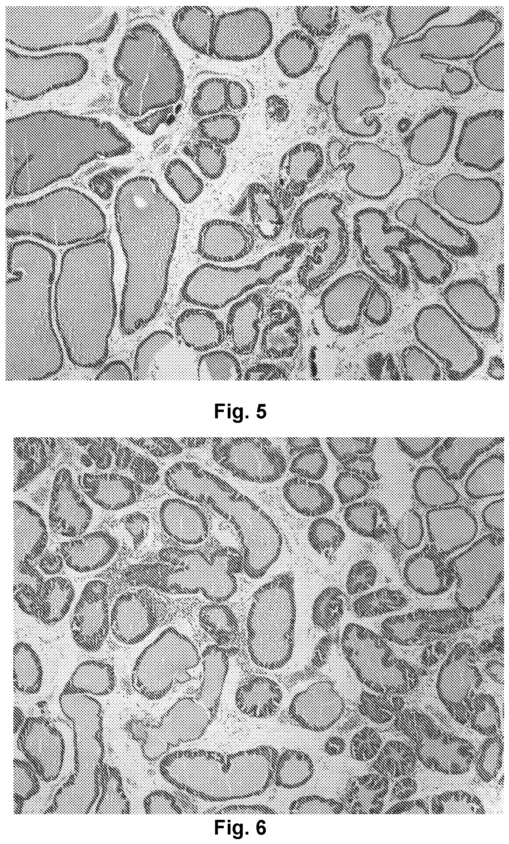

Mouse model for prostatitis was utilized to study the effects of DCP. After treatment with a DCP composition for 1-2 weeks, mice suffering from nonbacterial prostatitis returned to normal. The results are shown in Fig. 3. - Fig. 5, which demonstrate *in vivo* pathology findings for treating prostatitis with DCP.

As shown in Fig. 3, prostate tissues from the control group of mice showed no granulomatous lesions and there was a small amount of interstitial infiltration of inflammatory cells. Fig. 4 shows the tissues from mice suffering from prostatitis without treatment. As demonstrated in Fig. 4, granulomatous lesions were observed inside the prostate tissues, visible foreign cystalline was found in the glandular cavity, and interstitial inflammatory cell infiltration was detected.

Fig. 5 shows the prostate tissues of a prostatitis mouse after treatment with DCP for one week. As shown in Fig. 5, granulomatous lesions were significantly reduced in some tissues or eliminated in others; the number of inflammatory cells in interstitial infiltration was significantly reduced.

The results with the mouse model demonstrate that DCP is effective in the treatment of prostatitis. Based on the effective amount of DCP in mice, the effective amount of DCP for human should range from 1 to 10 mg/Kg body weight.

### Studies on BPH

In a study of DCP in the male patients (human) suffering from cancers and simultaneously from benign prostatic hyperplasia (BPH), the symptoms of BPH were significantly alleviated with DCP treatment. The majority of the patients suffering BPH improved their prostate activity after two weeks of use of the DCP, such as the enlargement of prostate obviously inhibited, PSA decreased, maximum of urinary volume increased to more than 12 mL/s.

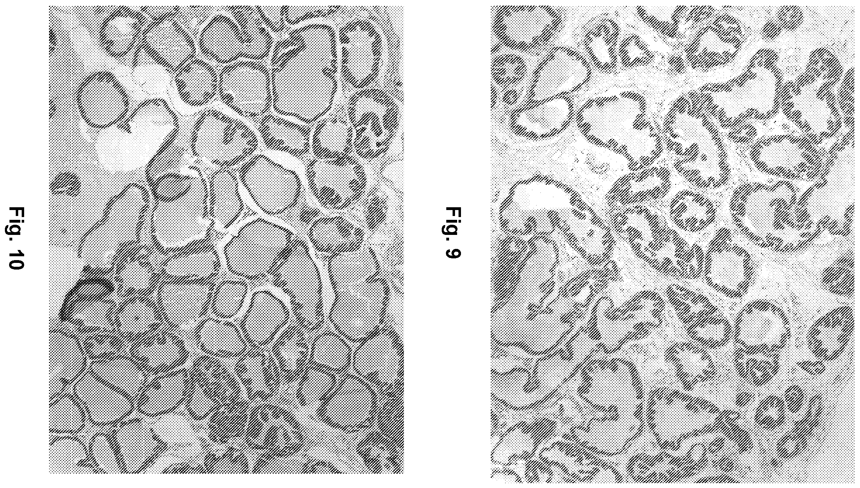

In a mouse model of BPH, treat with DCP resulted in the reduction of symptoms such as white cell number and lecithin density. The results are shown in Fig. 9, Fig. 10 and Table 1.

**Table 1. Effect of DCP on the number of white cell and density of lecithin**

| Groups | White cell (number/µL) | Lecithin corpuscle (density) |
|---|---|---|
| Control | 700 ± 13 | 38 ± 0.53 |
| BPH Model | 13005 ± 290 | 14 ± 0.53 |
| BPH + DCP Treated (injection) | 3250 ± 65 | 30 ± 0.53 |
| BPH + DCP Treated (Oral) | 3500 ± 71 | 27 ± 0.50 |

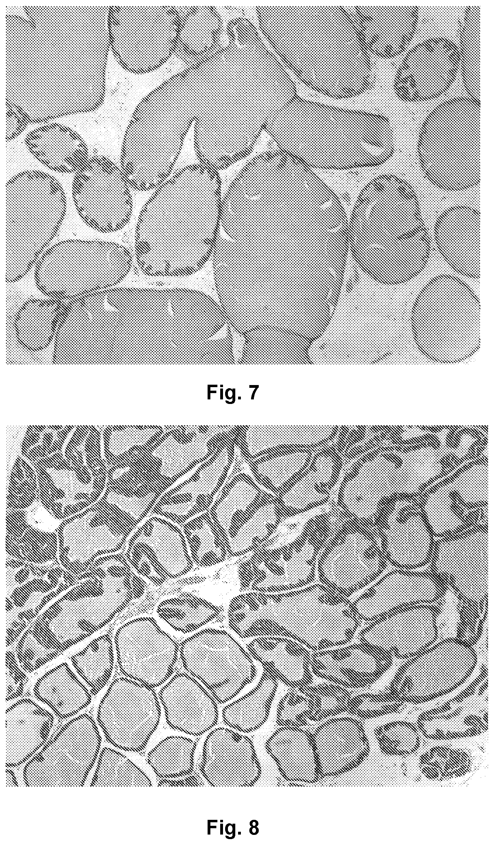

As shown in Fig. 6, a low level of lymphocytes and plasma cells were observed. In the BPH model group (Fig. 7), glands proliferation and a small amount of lymphocytes and plasma cells were shown. In the PROSCAR drug group (Fig. 8), glandular proliferation was not clear, but inflammation cells were reduced. In the DCP drug group (Fig. 9) (intravenous administration), inflammatory cells were significantly reduced, and glandular proliferation was inhibited, also as shown in Fig. 10 (oral administration), inflammatory cells were significantly reduced and glandular proliferation was inhibited.

Table 1 shows that enlargement of prostate was significant inhibited after treatment with DCP for a therapeutic period.

When at least one additional therapeutic agent or adjuvant therapy agent is used in combination with DCP, such as folic acid, coenzyme Q10, curcumin, glutathione (GSH), aloe vera, oryzanol, 5-fluorouracil, bortezomib, or any combination thereof, the treatment is more effective for the the alleviation of BHP, especially when using DCP injection solution. 5-fluorouracil is favorably combined with DCP to treat BPH.

Since BPH often leads to prostate cancer, treating BPH essentially prevents prostate cancer. Therefore, the methods herein disclosed to treat BPH with DCP also applies as methods to prevent prostate cancer.

## Claims

1. A pharmaceutical composition comprising dicycloplatin (DCP) for use in a method of treating or preventing a disease in a subject, wherein the disease is selected from the group consisting of rheumatoid arthritis, gout, lupus, osteoporosis, psoriasis, prostatitis, benign prostatic hyperplasia (BPH), multiple sclerosis, coeliac disease, Sjogren gren's syndrome, Hashimoto's thyroiditis, Graves' disease and graft-versus-host disease (GVHD).

2. The pharmaceutical composition for use of claim 1, wherein the disease is rheumatoid arthritis.

3. The pharmaceutical composition for use of claim 1, wherein the disease is prostatitis.

4. The pharmaceutical composition for use of claim 1, wherein the disease is benign prostatic hyperplasia (BPH).

5. The pharmaceutical composition for use of any one of claims 1-4, wherein the pharmaceutical composition further comprises at least one therapeutic agent or one adjuvant therapy agent.

6. The pharmaceutical composition for use of claim 5, wherein the at least one therapeutic agent or one adjuvant therapy agent is selected from the group consisting of folic acid, coenzyme Q10, curcumin, glutathione (GSH), aloe vera, oryzanol, 5-fluorouracil, and bortezomib.

7. The pharmaceutical composition for use of any one of claims 1-6, wherein the pharmaceutical composition further comprises a pharmaceutically acceptable carrier or excipient.

8. The pharmaceutical composition for use of any one of claims 1-7, wherein the pharmaceutical composition is administered via oral, buccal, inhalation spray, sublingual, rectal, transdermal, vaginal, transmucosal, topical, nasal, intestinal; intramuscular, subcutaneous, intramedullary, intrathecal, intraventricular, orthotopic, intrademal,, intraperitoneal, intravenous, intra-articular, intra-sternal, intra-synovial, intra-hepatic, intralesional, intracranial, intraperitoeal, intranasal, or intraocular routes.

9. The pharmaceutical composition for use of any one of claims 1-7, wherein DCP modulates the immunological reaction of the subject.

10. The pharmaceutical composition for use of any one of claims 1-7, wherein the amount of DCP in the pharmaceutical composition administered to the subject is at an amount of about 0.01 to 10 mg/kg body weight.

11. The pharmaceutical composition for use of any one of claims 1-7, wherein the pharmaceutical composition is administered via injection or oral route.

## Patentansprüche

1. Pharmazeutische Zusammensetzung umfassend Dicycloplatin (DCP) zur Verwendung in einem Verfahren zur Behandlung oder Vorbeugung einer Krankheit in einem Subjekt, wobei die Krankheit ausgewählt ist aus der Gruppe bestehend aus rheumatoider Arthritis, Gicht, Lupus, Osteoporose, Psoriasis, Prostatitis, benigner Prostatahyperplasie (BPH), multipler Sklerose, Zöliakie, Sjogren-Syndrom, Hashimoto-Thyreoiditis, Morbus Basedow und Graft-versus-Host-Reaktion (GVHD).

2. Pharmazeutische Zusammensetzung zur Verwendung nach Anspruch 1, wobei die Krankheit rheumatoide Arthritis ist.

3. Pharmazeutische Zusammensetzung zur Verwendung nach Anspruch 1, wobei die Krankheit Prostatitis ist.

4. Pharmazeutische Zusammensetzung zur Verwendung nach Anspruch 1, wobei die Krankheit benigne Prostatahyperplasie (BPH) ist.

5. Pharmazeutische Zusammensetzung zur Verwendung nach einem der Ansprüche 1-4, wobei die pharmazeutische Zusammensetzung ferner zumindest einen therapeutischen Wirkstoff oder ein therapeutisches Adjuvans umfasst.

6. Pharmazeutische Zusammensetzung zur Verwendung nach Anspruch 5, wobei der zumindest eine therapeutische Wirkstoff oder das eine therapeutische Adjuvans ausgewählt ist aus der Gruppe bestehend aus Folsäure, Coenzym Q10, Curcumin, Glutathion (GSH), Aloe vera, Oryzanol, 5-Fuorouracil und Bortezomib.

7. Pharmazeutische Zusammensetzung zur Verwendung nach einem der Ansprüche 1-6, wobei die pharmazeutische Zusammensetzung ferner einen pharmazeutisch akzeptablen Träger oder Exzipienten umfasst.

8. Pharmazeutische Zusammensetzung zur Verwendung nach einem der Ansprüche 1-7, wobei die pharmazeutische Zusammensetzung über den oralen, bukkalen Weg, als Inhalationsspray, den sublingualen, rektalen, transdermalen, vaginalen, transmukosalen, topischen, nasalen, intestinalen Weg; den intramuskulären, subkutanen, intramedullären, intrathekalen, intraventrikulären, orthotopischen, intradermalen, intraperitonealen, intravenösen, intraartikulären, intrasternalen, intrasynovialen, intrahepatischen, intraläsionalen, intrakranialen, intraperitonealen, intranasalen oder intraokularen Weg verabreicht wird.

9. Pharmazeutische Zusammensetzung zur Verwendung nach einem der Ansprüche 1-7, wobei DCP die immunologische Reaktion des Subjekts moduliert.

10. Pharmazeutische Zusammensetzung zur Verwendung nach einem der Ansprüche 1-7, wobei die Menge an DCP in der pharmazeutischen Zusammensetzung, die an das Subjekt verabreicht wird, eine Menge von etwa 0,01 bis 10 mg/kg Körpergewicht ist.

11. Pharmazeutische Zusammensetzung zur Verwendung nach einem der Ansprüche 1-7, wobei die pharmazeutische Zusammensetzung als Injektion oder auf oralem Weg verabreicht wird.

## Revendications

1. Composition pharmaceutique comprenant le dicycloplatine (DCP) pour une utilisation dans un procédé de traitement ou de prévention d'une maladie chez un sujet, où la maladie est choisie dans le groupe constitué de la polyarthrite rhumatoïde, de la goutte, du lupus, de l'ostéoporose, du psoriasis, de la prostatite, de l'hypertrophie bénigne de la prostate (HBP), de la sclérose en plaques, de la maladie cœliaque, du syndrome de Sjogren gren, de la thyroïdite de Hashimoto, de la maladie de Graves et de la réaction de greffe contre hôte (GVHD).

2. Composition pharmaceutique pour une utilisation selon la revendication 1, dans laquelle la maladie est la polyarthrite rhumatoïde.

3. Composition pharmaceutique pour une utilisation selon la revendication 1, dans laquelle la maladie est la prostatite.

4. Composition pharmaceutique pour une utilisation selon la revendication 1, dans laquelle la maladie est l'hypertrophie bénigne de la prostate (HBP).

5. Composition pharmaceutique pour une utilisation selon l'une quelconque des revendications 1 à 4, dans laquelle la composition pharmaceutique comprend en outre au moins un agent thérapeutique ou un agent de thérapie adjuvante.

6. Composition pharmaceutique pour une utilisation selon la revendication 5, dans laquelle l'au moins un agent thérapeutique ou un agent de thérapie adjuvante est choisi dans le groupe constitué de l'acide folique, de la coenzyme Q10, de la curcumine, du glutathion (GSH), de l'aloe vera, de l'oryzanol, du 5-fluorouracile et du bortézomib.

7. Composition pharmaceutique pour une utilisation selon l'une quelconque des revendications 1 à 6, dans laquelle la composition pharmaceutique comprend en outre un support ou excipient pharmaceutiquement acceptable.

8. Composition pharmaceutique pour une utilisation selon l'une quelconque des revendications 1 à 7, dans laquelle la composition pharmaceutique est administrée par voie orale, voie buccale, par aérosol d'inhalation, voie sublinguale, rectale, transdermique, vaginale, transmuqueuse, topique, nasale, intestinale ; intramusculaire, sous-cutanée, intramédullaire, intrathécale, intraventriculaire, orthotopique, intradermique, intrapéritonéale, intraveineuse, intra-articulaire, intra-sternale, intra-synoviale, intra-hépatique, intralésionnelle, intracrânienne, intrapéritonéale, intranasale ou intra-oculaire.

9. Composition pharmaceutique pour une utilisation selon l'une quelconque des revendications 1 à 7, dans laquelle DCP module la réaction immunologique du sujet.

10. Composition pharmaceutique pour une utilisation selon l'une quelconque des revendications 1 à 7, dans laquelle la quantité de DCP dans la composition pharmaceutique administrée au sujet est une quantité d'environ 0,01 à 10 mg/kg de poids corporel.

11. Composition pharmaceutique pour une utilisation selon l'une quelconque des revendications 1 à 7, dans laquelle la composition pharmaceutique est administrée par injection ou voie orale.
